# EUROPÄISCHE PATENTSCHRIFT

(11) **EP 1 051 404 B1**
(45) Veröffentlichungstag und Bekanntmachung des Hinweises auf die Patenterteilung: **16.04.2003**
(21) Anmeldenummer: 99906139.3
(22) Anmeldetag: 16.01.1999
(51) Int. Cl.: C07D 239/54, A01N 43/54

(54) **SUBSTITUIERTE PHENYLURACILE MIT HERBIZIDER WIRKUNG**
SUBSTITUTED PHENYLURACILS WITH HERBICIDAL EFFECT
PHENYLURACILES SUBSTITUES A EFFET HERBICIDE

(30) Priorität: 29.01.1998 DE 19803396
(43) Veröffentlichungstag der Anmeldung: 15.11.2000
(73) Patentinhaber: Bayer CropScience AG, 40789 Monheim (DE)
(72) Erfinder: ANDREE, Roland, D-40764 Langenfeld (DE); DOLLINGER, Markus, Overland Park, KS 66213 (US); DREWES, Mark, Wilhelm, D-40764 Langenfeld (DE); WETCHOLOWSKY, Ingo, D-51371 Leverkusen (DE); MYERS, Randy, Allen, D-40489 Düsseldorf (DE)
(86) Internationale Anmeldenummer: EP9900237
(87) Internationale Veröffentlichungsnummer: WO99038851

(56) Entgegenhaltungen:
- WO-A-95/17096

## Beschreibung

Die Erfindung betrifft neue substituierte Phenyluracile, Verfahren zu ihrer Herstellung und ihre Verwendung als Herbizide.

Eine große Zahl von substituierten Aryluracilen ist bereits aus der (Patent-)Literatur bekannt (vgl. US-A-5 399 543, WO-A-95 17 096) Diese Verbindungen haben jedoch bisher keine besondere Bedeutung erlangt.

Es wurden nun neue substituierte Phenyluracile der allgemeinen Formel (I) 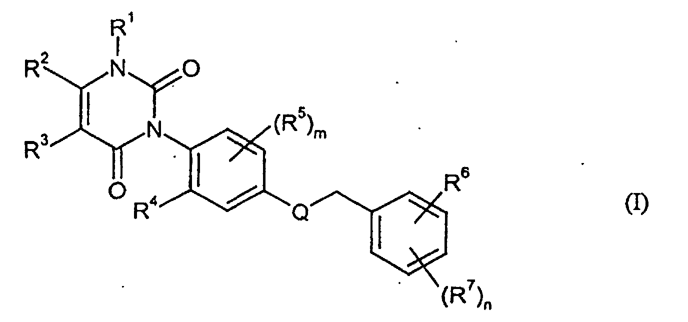 in welcher
- m: für die Zahlen 0, 1 oder 2 steht,
- n: für die Zahlen 0, 1, 2 oder 3 steht,
- Q: für 0, S, SO, SO₂, NH oder N(C₁-C₄-Alkyl) steht,
- R¹: für Wasserstoff, Amino oder gegebenenfalls durch Cyano, Halogen oder C₁-C₄-Alkoxy substituiertes Alkyl mit 1 bis 6 Kohlenstoffatomen steht,
- R²: für Carboxy, Cyano, Carbamoyl, Thiocarbamoyl, oder für jeweils gegebenenfalls durch Cyano, Halogen oder C₁-C₄-Alkoxy substituiertes Alkyl oder Alkoxycarbonyl mit jeweils bis zu 6 Kohlenstoffatomen steht,
- R³: für Wasserstoff, Halogen oder gegebenenfalls durch Cyano, Halogen oder C₁-C₄-Alkoxy substituiertes Alkyl mit 1 bis 6 Kohlenstoffatomen steht,
- R⁴: für Wasserstoff, Cyano oder Halogen steht,
- R⁵: für Nitro, Amino, Hydroxy, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino oder Alkylsulfonylamino mit jeweils bis zu 6 Kohlenstoffatomen steht, und
- R⁶: für Amino oder für eine der nachstehenden Gruppierungen
-NH-R⁸, -N(R⁸)₂, -NH-SO₂-R⁸, -N(R⁸)(SO₂-R⁸), -N(SO₂-R⁸)₂,
-NH-CO-R⁹, -N(R⁸)(CO-R⁹), -N(CO-R⁹)₂, -N(SO₂-R⁸)(CO-R⁹) steht,
- R⁷: für Nitro, Amino, Hydroxy, Mercapto, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino, Alkylsulfonylamino oder Bis-alkylsulfonyl-amino mit jeweils bis zu 6 Kohlenstoffatomen steht,
- R⁸: für gegebenenfalls durch Halogen substituiertes Alkyl mit 1 bis 6 Kohlenstoffatomen, für gegebenenfalls durch Halogen substituiertes Alkenyl mit 2 bis 6 Kohlenstoffatomen, für gegebenenfalls durch Cyano, Halogen oder C₁-C₄-Alkyl substituiertes Cycloalkyl mit 3 bis 6 Kohlenstoffatomen, für jeweils gegebenenfalls durch Nitro, Cyano, Thiocarbamoyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für gegebenenfalls durch Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl substituiertes Heterocyclyl aus der Reihe Furyl, Thienyl, Oxazolyl, Isoxazolyl, Pyrazolyl, Pyridyl, Pyrimidyl steht, und
- R⁹: für Wasserstoff, für jeweils gegebenenfalls durch Cyano, Halogen oder C₁-C₄-Alkoxy substituiertes Alkyl, Alkoxy, Alkylamino oder Dialkylamino mit jeweils 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Nitro, Cyano, Thiocarbamoyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxy-carbonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für gegebenenfalls durch Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl substituiertes Heterocyclyl aus der Reihe Furyl, Thienyl, Oxazolyl, Isoxazolyl, Pyrazolyl, Pyridyl, Pyrimidyl steht,
mit Ausnahme der vorbekannten Verbindungen 1-[4-(4-Nitro-phenylmethoxy-phenyl)]-3-methyl-4-trifluormethyl-3,6-dihydro-2,6-dioxo-1(2H)-pyrimidin und 1-[2-Fluor-4-(4-nitro-phenylmethoxy-phenyl)]-3-methyl-4-trifluorrnethyl-3,6-dihydro-2,6-dioxo-1(2H)-pyrimidin (vgl. US-A-5 399 543 und WO-A-95 17 096)
gefunden.

In den Definitionen sind die Kohlenwasserstoffketten, wie Alkyl - auch in Verbindung mit Heteroatomen, wie in Alkoxy -jeweils geradkettig oder verzweigt.

Die Erfindung betrifft insbesondere Verbindungen der Formel (I), in welcher
- m: für die Zahlen 0, 1 oder 2 steht,
- n: für die Zahlen 0, 1 oder 2 steht,
- Q: für O, S, SO, SO₂, NH oder N(CH₃) steht,
- R¹: für Wasserstoff, Amino oder für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
- R²: für Carboxy, Cyano, Carbamoyl, Thiocarbamoyl, oder für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxycarbonyl, Ethoxycarbonyl, n- oder i-Propoxycarbonyl steht,
- R³: für Wasserstoff, Fluor, Chlor, Brom, oder für jeweils gegebenenfalls durch Cyano, Fluor, Chlor, Methoxy oder Ethoxy substituiertes Methyl, Ethyl, n-oder i-Propyl, n-, i-, s- oder t-Butyl steht,
- R⁴: für Wasserstoff, Cyano, Fluor oder Chlor steht,
- R⁵: für Nitro, Amino, Hydroxy, Carboxy, Cyano, Carbamoyl, Thiocarbamoyl, Sulfo, Chlorsulfonyl, Aminosulfonyl, Fluor, Chlor, Brom, oder für jeweils gegebenenfalls druch Cyano, Fluor, Chlor, Methoxy oder Ethoxy substituiertes Methyl, Ethyl, n- oder i-Propyl, n-, i-, s- oder t-Butyl, Methoxy, Ethoxy, n-oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Acetylamino, Propionylamino, n-oder i-Butyroylamino, Methoxycarbonylamino, Ethoxycarbonylamino, n-oder i-Propoxycarbonylamino, Methylsulfonylamino, Ethylsulfonylamino, n-oder i-Propylsulfonylamino, n-, i-, s- oder t-Butylsulfonylamino steht, und
- R⁶: für Amino oder für eine der nachstehenden Gruppierungen
-NH-R⁸, -N(R⁸)₂, -NH-SO₂-R⁸, -N(R⁸)(SO2-R⁸), -N(SO₂-R⁸)₂,
-NH-CO-R⁹, -N(R⁸)(CO-R⁹), -N(CO-R⁹)₂, -N(SO₂-R⁸)(CO-R⁹) steht,
- R⁷: für Nitro, Amino, Hydroxy, Mercapto, Carboxy, Cyano, Carbamoyl, Thiocarbamoyl, Sulfo, Chlorsulfonyl, Aminosulfonyl, Fluor, Chlor, Brom, oder fiir jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-oder i-Propylthio, n-, i-, s- oder t-Butylthio, Methylsulfinyl, Ethylsulfinyl, n-oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, n-, i-, s- oder t-Butylsulfonylamino, Bis-(methylsulfonyl)-amino oder Bis-(ethylsulfonyl)-amino steht,
- R⁸: für jeweils gegebenenfalls durch Fluor und/oder Chlor substituiertes Methyl, Ethyl, n- oder i-Propyl, n-, i-, s- oder t-Butyl; für jeweils gegebenenfalls durch Fluor und/oder Chlor substituiertes Ethenyl, Propenyl oder Butenyl, für jeweils gegebenenfalls durch Cyano, Fluor, Chlor, Methyl oder Ethyl substituiertes Cyclopropyl, Cyclobutyl, Cyclopentyl oder Cyclohexyl, für jeweils gegebenenfalls durch Nitro, Cyano, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Methoxycarbonyl oder Ethoxy-carbonyl substituiertes Phenyl oder Benzyl, oder für gegebenenfalls durch Cyano, Fluor, Chlor, Brom, Methyl, Ethyl, n- oder i-Propyl n-, i-, s-oder t-Butyl, Trifluormethyl, Methoxy, Ethoxy, n- oder i-Propoxy, n-, i-, s-oder t-Butoxy, Difluormethoxy, Trifluormethoxy, Methylthio, Ethylthio, n-oder i-Propylthio, n-, i-, s- oder t-Butylthio, Trifluormethylthio, Methylsulfinyl, Ethylsulfinyl, Trifluormethylsulfinyl, Methylsulfonyl, Ethylsulfonyl, oder Trifluormethylsulfonyl substituiertes Heterocyclyl aus der Reihe Furyl, Thienyl, Oxazolyl, Isoxazolyl, Pyrazolyl, Pyridyl, Pyrimidyl steht, und
- R⁹: für Wasserstoff, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino oder Dimethylamino, für jeweils gegebenenfalls durch Cyano, Fluor oder Chlor substituiertes Ethenyl, Propenyl, Butenyl, Ethinyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Nitro, Cyano, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Methoxycarbonyl, Ethoxycarbonyl, n- oder i-Propoxy-carbonyl substituiertes Phenyl oder Benzyl, oder für gegebenenfalls durch Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Heterocyclyl aus der Reihe Furyl, Thienyl, Oxazolyl, Isoxazolyl, Pyrazolyl, Pyridyl, Pyrimidyl steht,
mit Ausnahme der vorbekannten Verbindungen 1-[4-(4-Nitro-phenylmethoxy-phenyl)]-3-methyl-4-trifluormethyl-3,6-dihydro-2,6-dioxo-1(2H)-pyrimidin und 1-[2-Fluor-4-(4-nitro-phenylmethoxy-phenyl)]-3-methyl-4-trifluormethyl-3,6-dihydro-2,6-dioxo-1(2H)-pyrimidin (vgl. US-A-5 399 543 und WO-A-95 17 096).

Ganz besonders bevorzugt sind Verbindungen der Formel (I), in welcher
- m: für die Zahl 0 steht,
- n: für die Zahlen 0 oder 1 steht,
- Q: für O steht,
- R¹: für Wasserstoff, Amino oder Methyl steht,
- R²: für Trifluormethyl steht,
- R³: für Wasserstoff steht,
- R⁴: für Fluor oder Chlor steht,
- R⁶: für Amino oder für eine der nachstehenden Gruppierungen -NH-SO₂-R⁸ oder -N(SO₂-R⁸)(CO-R⁹) steht,
- R⁷: für Nitro, Cyano, Fluor, Chlor, Brom oder für gegebenenfalls durch Fluor substituiertes Methyl oder Methoxy, insbesondere in der 4-Position, steht,
- R⁸: für Methyl oder Ethyl steht, und
- R⁹: für Methyl, Ethyl, n- oder i-Propyl, n-, i-, s- oder t-Butyl oder Phenyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Die neuen substituierten Phenyluracile der allgemeinen Formel (I) zeichnen sich durch starke und selektive herbizide Wirksamkeit aus.

Man erhält die neuen substituierten Phenyluracile der allgemeinen Formel (I),
in welcher
- m, n, R¹, R², R³, R⁴, R⁵, R⁶ und R⁷: die oben angegebene Bedeutung haben, und
- Q: für O, S, NH oder N(C₁-C₄)-Alkyl steht,
wenn man
(a) Phenyluracile der allgemeinen Formel (II) 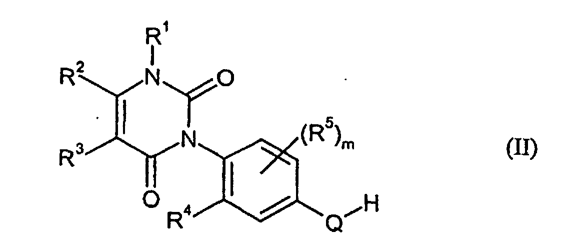
in welcher
- m, R¹, R², R³, R⁴ und R⁵: die oben angegebene Bedeutung haben, und
- Q: für O, S, NH oder N(C₁-C₄)-Alkyl steht,
mit substituierten Benzylhalogeniden der allgemeinen Formel (III) 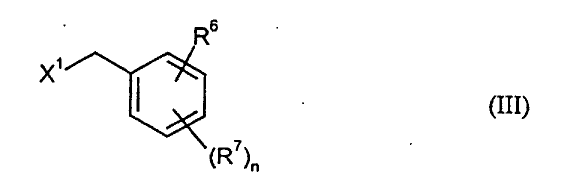 in welcher
- n, R⁶ und R⁷: die oben angegebene Bedeutung haben und
- X¹: für Halogen steht,
gegebenenfalls in Gegenwart eines Reaktionshilfsmittels und gegebenenfalls in Gegenwart eines Verdünnungsmittels umsetzt,
oder wenn man
(b) substituierte Phenyluracile der allgemeinen Formel (Ia) 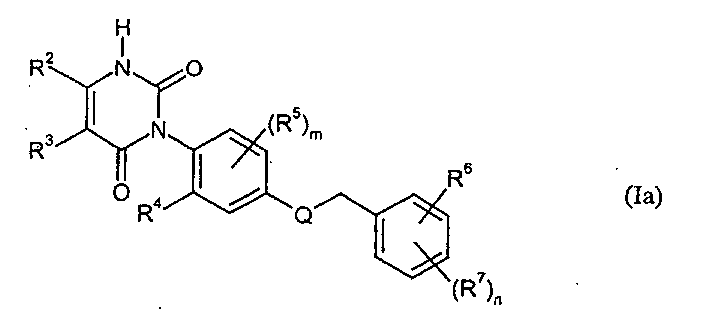
in welcher
- m, n, R², R³, R⁴, R⁵, R⁶ und R⁷: die oben angegebene Bedeutung haben, und
- Q: für O, S, NH oder N(C₁-C₄)-Alkyl steht,
mit 1-Aminooxy-2,4-dinitro-benzol oder mit Alkylierungsmitteln der allgemeinen Formel (IV)

X²-A¹ (IV)

in welcher
- A¹: für gegebenenfalls substituiertes C₁-C₆-Alkyl steht und
- X²: für Halogen oder die Gruppierung -O-S0₂-O-A¹ steht,
gegebenenfalls in Gegenwart eines Reaktionshilfsmittels und gegebenenfalls in Gegenwart eines Verdünnungsmittels umsetzt,
und gegebenenfalls im Anschluß daran im Rahmen der Substituentendefinition auf übliche Weise elektrophile oder nucleophile bzw. Oxidations- oder Reduktionsreaktionen durchführt.

Die beim erfindungsgemäßen Verfahren (a) zur Herstellung von Verbindungen der Formel (I) als Ausgangsstoffe zu verwendenden Phenyluracile sind durch die Formel (II) allgemein definiert. In der Formel (II) haben m, R¹, R², R³, R⁴ und R⁵ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für m, R¹, R², R³, R⁴ und R⁵ angegeben wurden.

Die Ausgangsstoffe der allgemeinen Formel (II) sind bekannt und/oder können nach an sich bekannten Verfahren hergestellt werden (vgl. EP-A-545 206, JP-A-04 178 373, Herstellungsbeispiele).

Die beim erfindungsgemäßen Verfahren (a) weiter als Ausgangsstoffe zu verwendenden substituierten Benzylhalogenide sind durch die Formel (III) allgemein definiert. In der Formel (III) haben n, R⁶ und R⁷ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, R⁶ und R⁷ angegeben wurden; X¹ steht vorzugsweise für Fluor, Chlor, Brom oder Iod, insbesondere für Chlor oder Brom.

Die Ausgangsstoffe der allgemeinen Formel (III) sind bekannte organischen Synthesechemikalien.

Die beim erfindungsgemäßen Verfahren (b) zur Herstellung von Verbindungen der Formel (I) als Ausgangsstoffe zu verwendenden substituierten Phenyluracile sind durch die Formel (Ia) allgemein definiert. In der Formel (Ia) haben m, n, R², R³, R⁴, R⁵, R⁶ und R⁷ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für m, n, R², R³, R⁴, R⁵, R⁶ und R⁷ angegeben wurden.

Die Ausgangsstoffe der allgemeinen Formel (Ia) für Verfahren (b) sind als neue Stoffe auch Gegenstand der vorliegenden Anmeldung; sie können nach dem erfindungsgemäßen Verfahren (a) hergestellt werden.

Die beim erfindungsgemäßen Verfahren (b) weiter als Ausgangsstoffe zu verwendenden Alkylierungsmittel sind durch die Formel (IV) allgemein definiert. In der Formel (IV) stehen vorzugsweise A¹ für gegebenenfalls durch Cyano, Halogen oder C₁-C₄-Alkoxy substituiertes Alkyl mit 1 bis 4 Kohlenstoffatomen und X² für Chlor, Brom, Iod, Methylsulfonyloxy oder Ethylsulfonyloxy; insbesondere stehen A¹ für jeweils gegebenenfalls durch Cyano, Fluor, Chlor, Methoxy oder Ethoxy substituiertes Methyl, Ethyl, n- oder i-Propyl und X² für Chlor, Brom, Iod, Methylsulfonyloxy oder Ethylsulfonyloxy.

Die Ausgangsstoffe der Formel (IV) sind bekannte organische Synthesechemikalien.

Die erfindungsgemäßen Verfahren zur Herstellung der Verbindungen der allgemeinen Formel (I) werden vorzugsweise unter Verwendung von Verdünnungsmitteln durchgeführt. Als Verdünnungsmittel zur Durchführung der erfindungsgemäßen Verfahren (a) und (b) kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder - diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Als Reaktionshilfsmittel für die erfindungsgemäßen Verfahren (a) und (b) kommen im allgemeinen die üblichen anorganischen oder organischen Basen oder Säureakzeptoren in Betracht. Hierzu gehören vorzugsweise Alkalimetall- oder Erdalkalimetall-acetate, -amide, -carbonate, -hydrogencarbonate, -hydride, -hydroxide oder - alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kaliumoder Calcium-hydrid, Lithium-, Natrium-, Kalium- oder Calcium-hydroxid, Natriumoder Kaliummethanolat, -ethanolat, -n- oder -i-propanolat, -n-, -i-, -s- oder -tbutanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Als weitere Reaktionshilfsmittel für die erfindungsgemäßen Verfahren (a) und (b) kommen auch Phasentransfer-Katalysatoren in Betracht. Als Beispiele für solche Katalysatoren seien genannt:
Tetrabutylammonium-bromid, Tetrabutylammonium-chlorid, Tetraoctylammoniumchlorid, Tetrabutylammonium-hydrogensulfat, Methyl-trioctylammonium-chlorid, Hexadecyl-trimethylammonium-chlorid, Hexadecyl-trimethylammonium-bromid, Benzyl-trimethylammonium-chlorid, Benzyl-triethylammonium-chlorid, Benzyl-trimethylammonium-hydroxid, Benzyl-triethylammonium-hydroxid, Benzyl-tributylammonium-chlorid, Benzyl-tributylammonium-bromid, Tetrabutylphosphoniumbromid, Tetrabutylphosphonium-chlorid, Tributyl-hexadecylphosphonium-bromid, Butyl-triphenylphosphonium-chlorid, Ethyl-trioctylphosphonium-bromid, Tetraphenylphosphonium-bromid.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0°C und 150°C, vorzugsweise zwischen 10°C und 120°C.

Die erfindungsgemäßen Verfahren werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvemichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlomitrofen, Chlorsulfuron, Chlortoluron, Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isouron, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargpnsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Terbuthylazine, Terbutryn, Thenylchlor, Thiafluamide, Thiazopyr, Thidiazimin, Thifensulfuron(-methyl),. Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aurwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

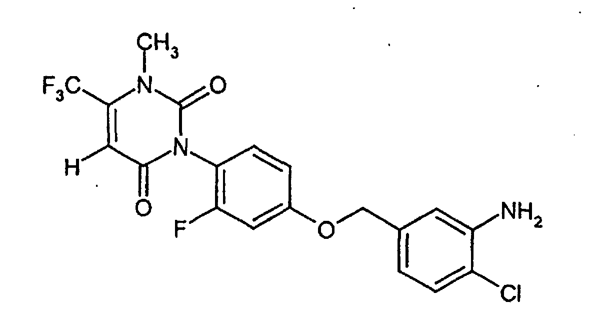

(Folgeumsetzung)
Eine Mischung aus 3,0 g (6,3 mMol) 1-[2-Fluor-4-(4-chlor-3-nitro-benzyloxy)-phenyl]-3-methyl-4-trifluormethyl-3,6-dihydro-2,6-dioxo-1(2H)-pyrimidin, 40 ml Essigsäure und 10 ml Wasser wird bei 50°C unter Rühren mit 1,8 g Eisen (-Pulver) portionsweise versetzt, dann 90 Minuten bei Raumtemperatur (ca. 20°C) gerührt und anschließend im Wasserstrahlvakuum eingeengt. Der Rückstand wird in Wasser aufgenommen und mit Essigsäureethylester extrahiert. Die organische Phase wird mit Wasser, 0,1%iger Natronlauge und 1%iger Salzsäure gewaschen, mit Natriumsulfat getrocknet und filtriert. Das Filtrat wird im Wasserstrahlvakuum eingeengt, der Rückstand mit Diethylether/Petrolether digeriert und das kristallin angefallene Produkt durch Absaugen isoliert.

Man erhält 2,2 g (78,5 % der Theorie) 1-[2-Fluor-4-(3-amino-4-chlor-benzyloxy)-phenyl]-3-methyl-4-trifluormethyl-3,6-dihydro-2,6-dioxo-1(2H)-pyrimidin vom Schmelzpunkt 148°C.

### Beispiel 2

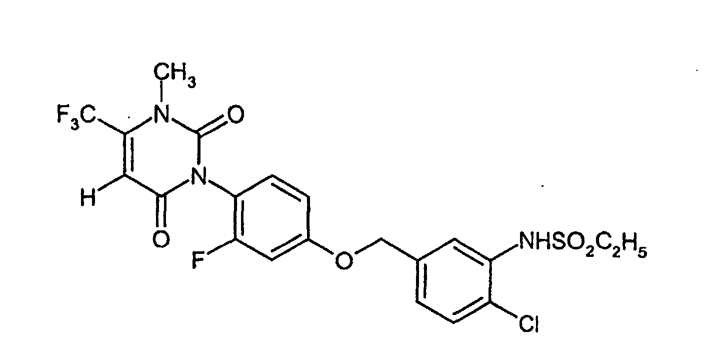

(Folgeumsetzung)
Eine Mischung aus 1,8 g (4,05 mMol) 1-[2-Fluor-4-(3-amino-4-chlor-benzyloxy)-phenyl]-3-methyl-4-trifluormethyl-3,6-dihydro-2,6-dioxo-1(2H)-pyrimidin, 0,35 g Pyridin, einer Spatelspitze 4-Dimethylamino-pyridin und 10 ml Acetonitril wird bei 50°C mit 0,65 g (5 mMol) Ethansulfonsäurechlorid versetzt und die Reaktionsmischung wird 2 Stunden unter Rückfluß erhitzt. Nach Zugabe von weiteren 0,5 g Pyridin und weiteren 0,65 g Ethansulfonsäurechlorid weitere 2 Stunden unter Rückfluß erhitzt und anschließend im Wasserstrahlvakuum eingeengt. Der Rückstand wird in 1%iger wässriger Natriumdihydrogenphosphat-Lösung aufgenommen und mit Essigsäureethylester extrahiert. Die organische Phase wird mit Wasser gewaschen, mit Natriumsulfat getrocknet und filtriert. Der Rückstand wird säulenchromatografisch (Kieselgel, Hexan/Essigsäureethylester, Vol.: 3:1) gereinigt.

Man erhält 0,60 g (28 % der Theorie) 1-[2-Fluor-4-(4-chlor-3-ethylsulfonylaminobenzyloxy)-phenyl]-3-methyl-4-trifluormethyl-3,6-dihydro-2,6-dioxo-1(2H)-pyrimidin vom Schmelzpunkt 151°C.

Analog zu den Herstellungsbeispielen 1 bis 5 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

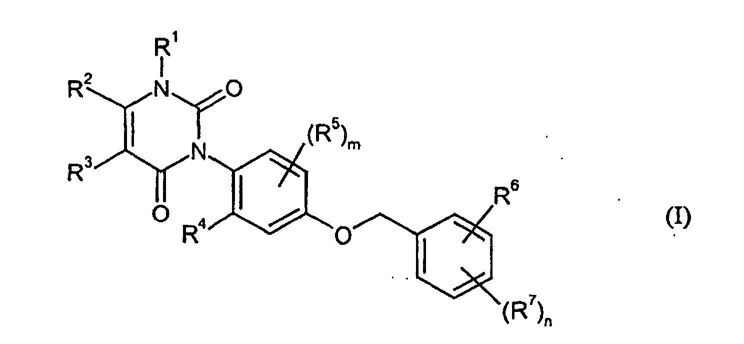

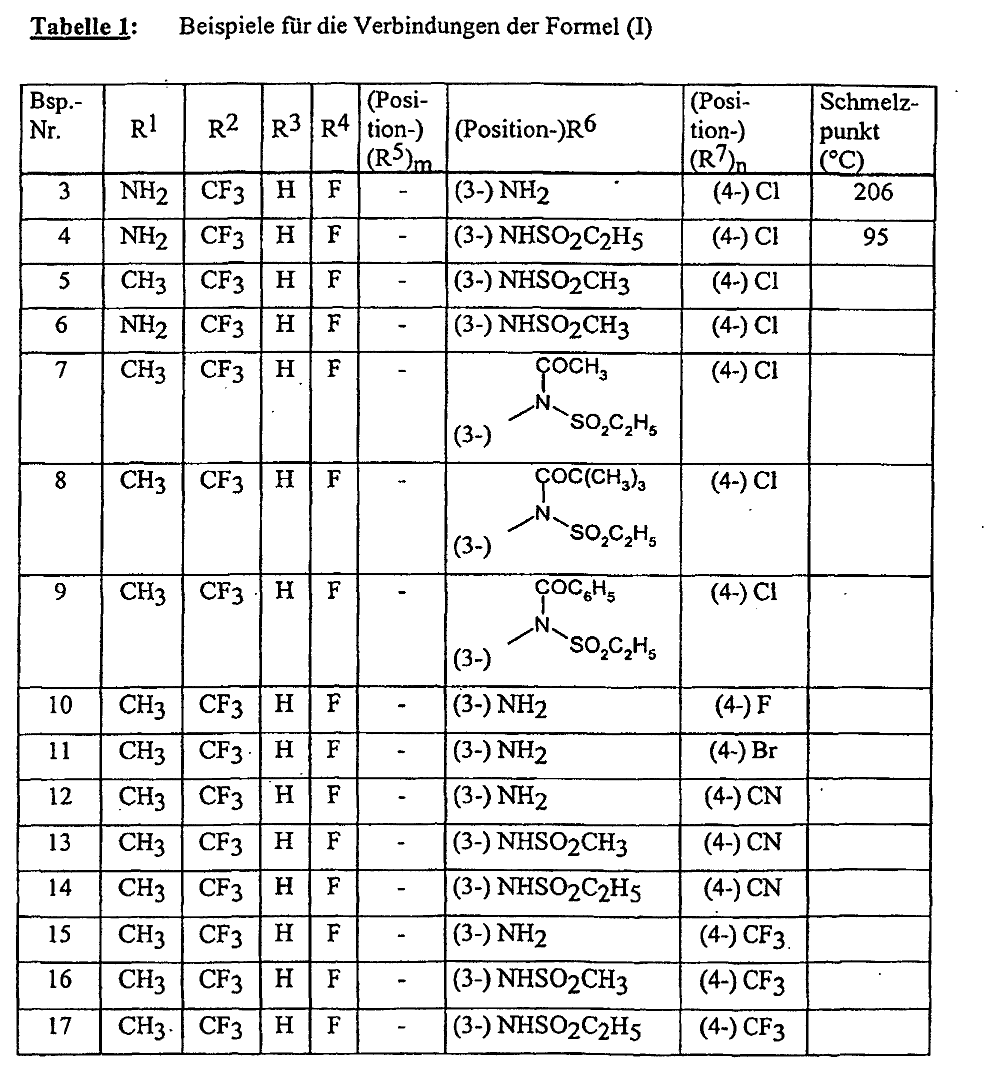

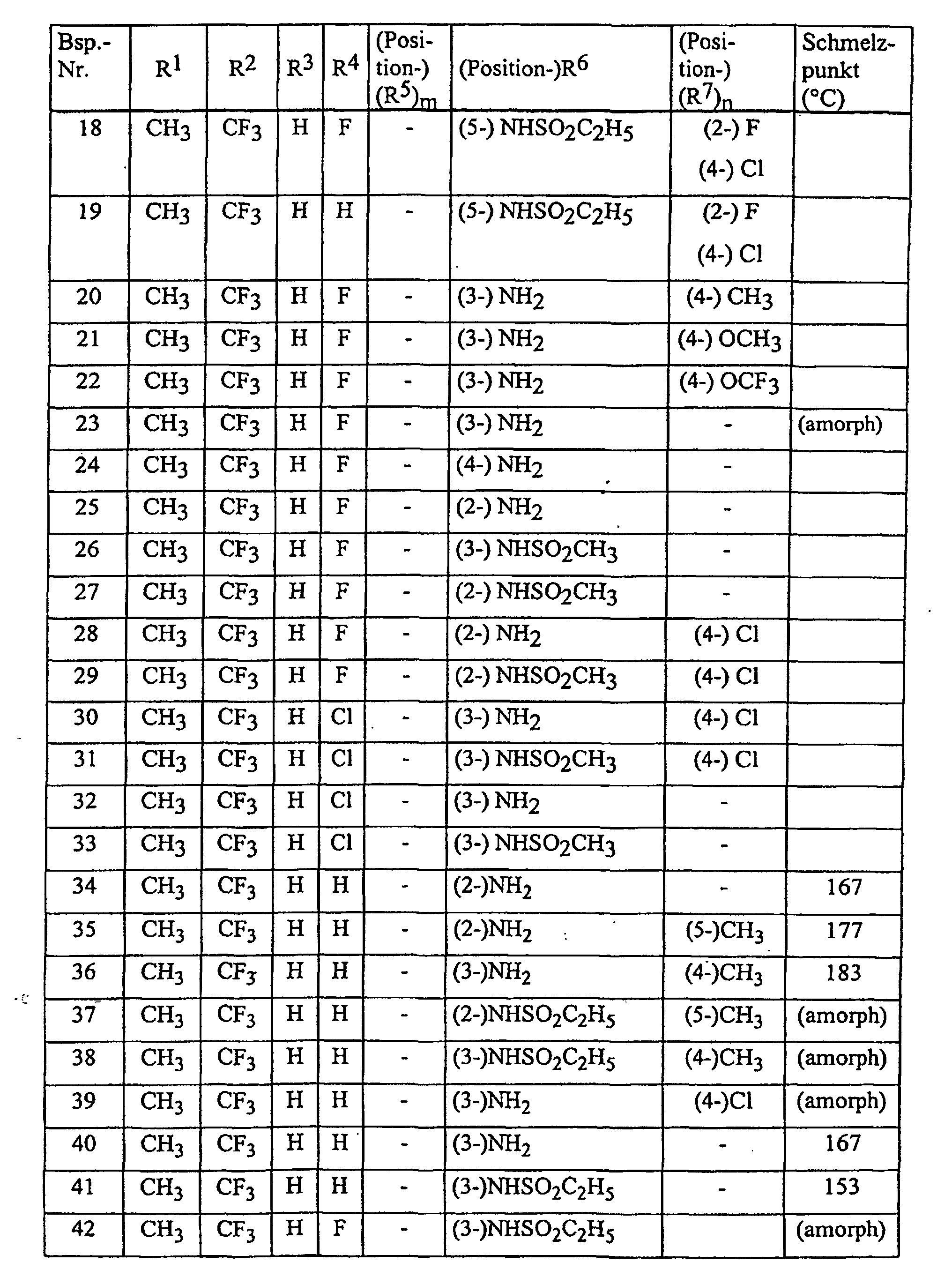

### Ausgangsstoffe der Formel (II):

### Beispiel (II-1)

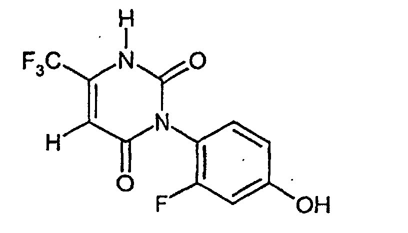

### Stufe 1

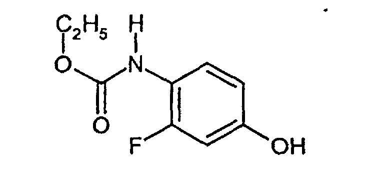

9,1 g (84 mMol) Chlorameisensäure-ethylester werden bei 0°C unter Rühren tropfenweise zu einer Mischung aus 9,8 g (77 mMol) 4-Amino-3-fluor-phenol, 2,3 g Magnesiumoxid, 150 ml Essigsäureethylester und 15 ml Wasser gegeben und die Reaktionsmischung wird 60 Minuten bei 0°C gerührt. Dann wird mit 1%iger wässriger Natriumdihydrogenphosphat-Lösung auf etwa das doppelte Volumen verdünnt, die organische Phase abgetrennt, mit Wasser gewaschen, mit Natriumsulfat getrocknet und filtriert. Das Filtrat wird im Wasserstrahlvakuum eingeengt, der Rückstand mit Diethylether/Petrolether digeriert und das kristallin angefallene Produkt durch Absaugen isoliert.

Man erhält 14,2 g (93 % der Theorie) N-(2-Fluor-4-hydroxy-phenyl)-O-ethyl-urethan vom Schmelzpunkt 111°C.

### Stufe 2

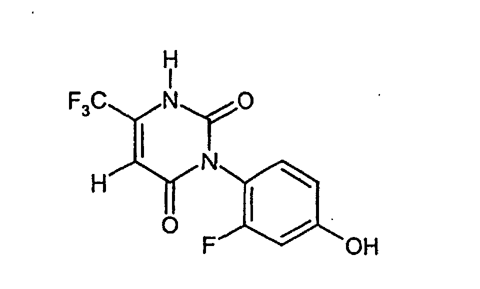

15,6 g (70 mMol) 3-Amino-4,4,4-trifluor-crotonsäure-ethylester werden in 50 ml N-Methyl-pyrrolidon vorgelegt und bei Raumtemperatur (ca. 20°C) mit 2,5 g Natriumhydrid versetzt. Die Mischung wird 30 Minuten bei Raumtemperatur gerührt und anschließend mit 14,0 g (70 mMol) N-(2-Fluor-4-hydroxy-phenyl)-O-ethyl-urethan versetzt. Die Reaktionsmischung wird 45 Minuten bei 140°C gerührt und dann nach leichtem Abkühlen auf etwa die gleiche Menge Eiswasser gegossen. Nach Schütteln mit Essigsäureethylester wird die wässrige Phase abgetrennt, mit 2N-Salzsäure angesäuert und mit Essigsäureethylester extrahiert. Die organische Phase wird mit Natriumsulfat getrocknet und filtriert. Das Filtrat wird im Wasserstrahlvakuum eingeengt, der Rückstand mit Diethylether digeriert und das kristallin angefallene Produkt durch Absaugen isoliert.

Man erhält 17,15 g (84 % der Theorie) 1-(2-Fluor-4-hydroxy-phenyl)-4-trifluormethyl-3,6-dihydro-2,6-dioxo-1(2H)-pyrimidin vom Schmelzpunkt 239°C.

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung so bespritzt, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 5 und 7 bei guter Verträglichkeit gegenüber Kulturpflanzen, wie z.B. Gerste und Baumwolle, sehr starke Wirkung gegen Unkräuter.

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 2 und 3 bei teilweise guter Verträglichkeit gegenüber Kulturpflanzen, wie z.B. Weizen, sehr starke Wirkung gegen Unkräuter.

## Patentansprüche

1. Substituierte Phenyluracile der allgemeinen Formel (I) 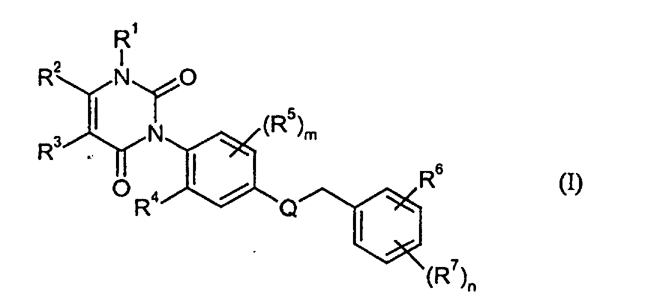 in welcher
m für die Zahlen 0, 1 oder 2 steht,
n für die Zahlen 0, 1, 2 oder 3 steht,
Q für O, S, SO, SO₂, NH oder N(C₁-C₄-Alkyl) steht,
R¹ für Wasserstoff, Amino oder gegebenenfalls durch Cyano, Halogen oder C₁-C₄-Alkoxy substituiertes Alkyl mit 1 bis 6 Kohlenstoffatomen steht,
R² für Carboxy, Cyano, Carbamoyl, Thiocarbamoyl, oder für jeweils gegebenenfalls durch Cyano, Halogen oder C₁-C₄-Alkoxy substituiertes Alkyl oder Alkoxycarbonyl mit jeweils bis zu 6 Kohlenstoffatomen steht,
R³ für Wasserstoff, Halogen oder gegebenenfalls durch Cyano, Halogen oder C₁-C₄-Alkoxy substituiertes Alkyl mit 1 bis 6 Kohlenstoffatomen steht,
R⁴ für Wasserstoff, Cyano oder Halogen steht,
R⁵ für Nitro, Amino, Hydroxy, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino oder Alkylsulfonylamino mit jeweils bis zu 6 Kohlenstoffatomen steht, und
R⁶ für Amino oder für eine der nachstehenden Gruppierungen
-NH-R⁸, -N(R⁸)₂, -NH-SO₂-R⁸, -N(R⁸)(SO2-R⁸), -N(SO₂-R⁸)₂,
-NH-CO-R⁹, N(R⁸)(CO-R⁹), -N(CO-R⁹)₂, -N(SO₂-R⁸)(CO-R⁹) steht,
R⁷ für Nitro, Amino, Hydroxy, Mercapto, Carboxy, Cyano, Carbamoyl, Thiocarbamoyl, Sulfo, Chlorsulfonyl, Aminosulfonyl, Halogen, oder für jeweils gegebenenfalls durch Cyano, Halogen oder C₁-C₄-Alkoxy substituiertes Alkyl, Alkoxy, Alkylthio, Alkylsulfinyl, Alkylsulfonyl, Alkylamino, Alkylcarbonyl, Alkoxycarbonyl, Alkylaminocarbonyl, Alkylcarbonylamino, Alkoxycarbonylamino, Alkylsulfonylamino oder Bis-alkylsulfonyl-amino mit jeweils bis zu 6 Kohlenstoffatomen steht,
R⁸ für gegebenenfalls durch Halogen substituiertes Alkyl mit 1 bis 6 Kohlenstoffatomen, für gegebenenfalls durch Halogen substituiertes Alkenyl mit 2 bis 6 Kohlenstoffatomen, für gegebenenfalls durch Cyano, Halogen oder C₁-C₄-Alkyl substituiertes Cycloalkyl mit 3 bis 6 Kohlenstoffatomen, für jeweils gegebenenfalls durch Nitro, Cyano, Thiocarbamoyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxycarbonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für gegebenenfalls durch Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl substituiertes Heterocyclyl aus der Reihe Furyl, Thienyl, Oxazolyl, Isoxazolyl, Pyrazolyl, Pyridyl, Pyrimidyl steht, und
R⁹ für Wasserstoff, für jeweils gegebenenfalls durch Cyano, Halogen oder C₁-C₄-Alkoxy substituiertes Alkyl, Alkoxy, Alkylamino oder Dialkylamino mit jeweils 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Nitro, Cyano, Thiocarbamoyl, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder C₁-C₄-Alkoxycarbonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für gegebenenfalls durch Cyano, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl substituiertes Heterocyclyl aus der Reihe Furyl, Thienyl, Oxazolyl, Isoxazolyl, Pyrazolyl, Pyridyl, Pyrimidyl steht,
mit Ausnahme der Verbindungen 1-[4-(4-Nitro-phenylmethoxy-phenyl)]-3-methyl-4-trifluormethyl-3,6-dihydro-2,6-dioxo-1(2H)-pyrimidin und 1-[2-Fluor-4-(4-mtro-phenylmethoxy-phenyl)]-3-methyl-4-trifluormethyl-3,6-dihydro-2,6-dioxo-1(2H)-pyrimidin.

2. Substituierte Phenyluracile gemäß Anspruch 1, **dadurch gekennzeichnet, dass**
m für die Zahlen 0, 1 oder 2 steht,
n für die Zahlen 0, 1 oder 2 steht,
Q für O, S, SO, SO₂, NH oder N(CH₃) steht,
R¹ für Wasserstoff, Amino oder für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
R² für Carboxy, Cyano, Carbamoyl, Thiocarbamoyl, oder für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxycarbonyl, Ethoxycarbonyl, n- oder i-Propoxycarbonyl steht,
R³ für Wasserstoff, Fluor, Chlor, Brom, oder für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
R⁴ für Wasserstoff, Cyano, Fluor oder Chlor steht,
R⁵ für Nitro, Amino, Hydroxy, Carboxy, Cyano, Carbamoyl, Thiocarbamoyl, Sulfo, Chlorsulfonyl, Aminosulfonyl, Fluor, Chlor, Brom, oder für jeweils gegebenenfalls druch Cyano, Fluor, Chlor, Methoxy oder Ethoxy substituiertes Methyl, Ethyl, n- oder i-Propyl, n-, i-, s-oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, n-, i-, s-oder t-Butylsulfonylamino steht, und
R⁶ für Amino oder für eine der nachstehenden Gruppierungen
-NH-R⁸, -N(R⁸)₂, -NH-SO₂-R⁸, -N(R⁸)(SO2-R⁸), -N(SO₂-R⁸)₂,
-NH-CO-R⁹, -N(R⁸)(CO-R⁹), -N(CO-R⁹)₂, -N(SO₂-R⁸)(CO-R⁹) steht,
R⁷ für Nitro, Amino, Hydroxy, Mercapto, Carboxy, Cyano, Carbamoyl, Thiocarbamoyl, Sulfo, Chlorsulfonyl, Aminosulfonyl, Fluor, Chlor, Brom, oder für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, n-, i-, s-oder t-Butylsulfonylamino, Bis-(methylsulfonyl)-amino oder Bis-(ethylsulfonyl)-amino steht,
R⁸ für jeweils gegebenenfalls durch Fluor und/oder Chlor substituiertes Methyl, Ethyl, n- oder i-Propyl, n-, i-, s- oder t-Butyl, für jeweils gegebenenfalls durch Fluor und/oder Chlor substituiertes Ethenyl, Propenyl oder Butenyl, für jeweils gegebenenfalls durch Cyano, Fluor, Chlor, Methyl oder Ethyl substituiertes Cyclopropyl, Cyclobutyl, Cyclopentyl oder Cyclohexyl, für jeweils gegebenenfalls durch Nitro, Cyano, Thiocarbamoyl, Fluor, Chlor, Brom, Methyl, Ethyl, n- oder i-Propyl, n-, i-, s- oder t-Butyl, Trifluormethyl, Methoxy, Ethoxy, n-oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Methoxycarbonyl oder Ethoxycarbonyl substituiertes Phenyl oder Benzyl, oder für gegebenenfalls durch Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, n-, i-, s- oder t-Butylthio, Trifluormethylthio, Methylsulfinyl, Ethylsulfinyl, Trifluormethylsulfinyl, Methylsulfonyl, Ethylsulfonyl, oder Trifluormethylsulfonyl substituiertes Heterocyclyl aus der Reihe Furyl, Thienyl, Oxazolyl, Isoxazolyl, Pyrazolyl, Pyridyl, Pyrimidyl steht, und
R⁹ für Wasserstoff, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s-oder t-Butylamino oder Dimethylamino, für jeweils gegebenenfalls durch Cyano, Fluor oder Chlor substituiertes Ethenyl, Propenyl, Butenyl, Ethinyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Nitro, Cyano, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n-oder i-Propylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Methoxycarbonyl, Ethoxycarbonyl, n- oder i-Propoxy-carbonyl substituiertes Phenyl oder Benzyl, oder für gegebenenfalls durch Cyano, Fluor, Chlor, Brom, Methyl, Ethyl, n-oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Heterocyclyl aus der Reihe Furyl, Thienyl, Oxazolyl, Isoxazolyl, Pyrazolyl, Pyridyl, Pyrimidyl steht,
mit Ausnahme der Verbindungen 1-[4-(4-Nitro-phenylmethoxy-phenyl)]-3-methyl-4-trifluormethyl-3,6-dihydro-2,6-dioxo-1(2H)-pyrimidin und 1-[2-Fluor-4-(4-nitro-phenylmethoxy-phenyl)]-3-methyl-4-trifluormethyl-3,6-dihydro-2,6-dioxo-1(2H)-pyrimidin.

3. Verfahren zum Herstellen von substituierten Phenyluracilen der Formel (I) gemäß einem der Ansprüche 1 oder 2,
in welcher
m, n. R¹, R², R³, R⁴, R⁵, R⁶ und R⁷ die in einem der Ansprüche 1 oder 2 angegebene Bedeutung haben, und Q für O, S, NH oder N(C₁-C₄-Alkyl) steht,
**dadurch gekennzeichnet, dass** man
(a) Phenyluracile der allgemeinen Formel (II) 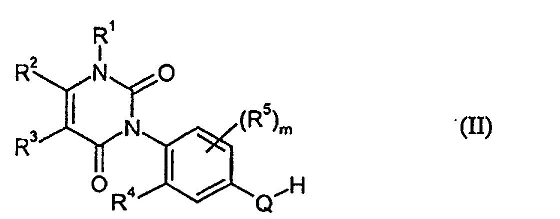
in welcher
m, R¹, R², R³, R⁴ und R⁵ die in einem der Ansprüche 1 oder 2 angegebene Bedeutung haben, und Q für Ö, S, NH oder N(C₁-C₄-Alkyl) steht
mit substituierten Benzylhalogeniden der allgemeinen Formel (III) 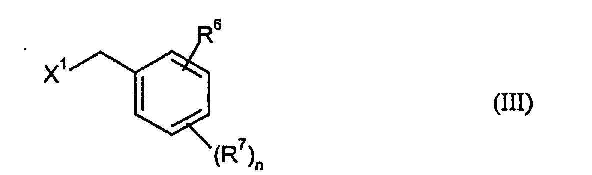 in welcher
n, R⁶ und R⁷ die in einem der Ansprüche 1 oder 2 angegebene Bedeutung haben und
X¹ für Halogen steht,
gegebenenfalls in Gegenwart eines Reaktionshilfsmittels und gegebenenfalls in Gegenwart eines Verdünnungsmittels umsetzt,
oder dass man
(b) substituierte Phenyluracile der allgemeinen Formel (Ia) 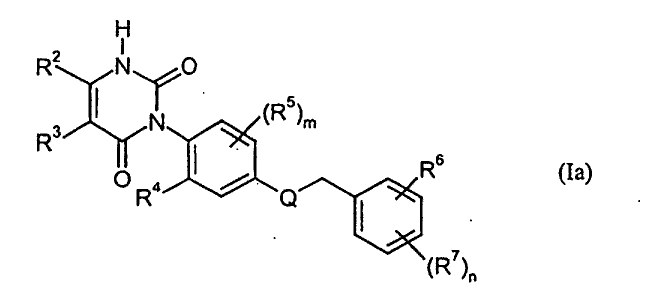
in welcher
m, n, Q, R², R³, R⁴, R⁵, R⁶ und R⁷ die oben angegebene Bedeutung haben,
mit 1-Aminooxy-2,4-dinitro-benzol oder mit Alkylierungsmitteln der allgemeinen Formel (IV)
X²-A¹ (IV)
in welcher
A¹ für gegebenenfalls substituiertes C₁-C₆-Alkyl steht und
X² für Halogen oder die Gruppierung -O-SO₂-O-A¹ steht,
gegebenenfalls in Gegenwart eines Reaktionshilfsmittels und gegebenenfalls in Gegenwart eines Verdünnungsmittels umsetzt,
und gegebenenfalls im Anschluß daran im Rahmen der Substituentendefinition auf übliche Weise elektrophile oder nucleophile bzw. Oxidationsoder Reduktionsreaktionen durchführt.

4. Substituierte Phenyluracile der allgemeinen Formel (Ia) 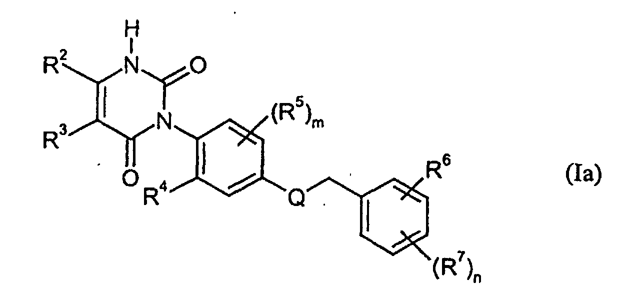 in welcher
m, n, Q, R², R³, R⁴, R⁵, R⁶ und R⁷ die in einem der Ansprüche 1 oder 2 angegebene Bedeutung haben.

5. Verwendung von mindestens einem substituierten Phenyluracil gemäß einem der Ansprüche 1 oder 2 zur Bekämpfung von unerwünschten Pflanzen.

6. Herbizides Mittel, **gekennzeichnet durch** den Gehalt an mindestens einem substituierten Phenyluracil gemäß einem der Ansprüche 1 oder 2 und üblichen Streckmitteln.

## Claims

1. Substituted phenyluracils of the general formula (I) 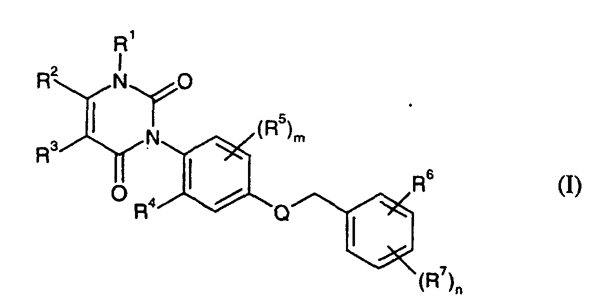 in which
m represents the numbers 0, 1 or 2,
n represents the numbers 0, 1, 2 or 3,
Q represents O, S, SO, SO₂,NH or N(C₁-C₄-alkyl),
R¹ represents hydrogen, amino or optionally cyano-, halogen- or C₁-C₄-alkoxy-substituted alkyl having 1 to 6 carbon atoms,
R² represents carboxyl, cyano, carbamoyl, thiocarbamoyl, or represents in each case optionally cyano-, halogen- or C₁-C₄-alkoxy-substituted alkyl or alkoxycarbonyl having in each case up to 6 carbon atoms,
R³ represents hydrogen, halogen or optionally cyano-, halogen- or C₁-C₄-alkoxy-substituted alkyl having 1 to 6 carbon atoms,
R⁴ represents hydrogen, cyano or halogen,
R⁵ represents nitro, amino, hydroxyl, carboxyl, cyano, carbamoyl, thiocarbamoyl, sulpho, chlorosulphonyl, aminosulphonyl, halogen, or represents in each case optionally cyano-, halogen- or C₁-C₄-alkoxysubstituted alkyl, alkoxy, alkylthio, alkylsulphinyl, alkylsulphonyl, alkylamino, alkylcarbonyl, alkoxycarbonyl, alkylaminocarbonyl, alkylcarbonylamino, alkoxycarbonylamino or alkylsulphonylamino having in each case up to 6 carbon atoms, and
R⁶ represents amino or one of the groupings below
-NH-R⁸, -N(R⁸)₂, -NH-SO₂-R⁸, -N(R⁸)(SO2-R⁸), -N(SO₂-R⁸)₂,
-NH-CO-R⁹, -N(R⁸)(CO-R⁹), -N(CO-R⁹)₂, -N(SO₂-R⁸)(CO-R⁹),
R⁷ represents nitro, amino, hydroxyl, mercapto, carboxyl, cyano, carbamoyl, thiocarbamoyl, sulpho, chlorosulphonyl, aminosulphonyl, halogen, or represents in each case optionally cyano-, halogen- or C₁-C₄-alkoxy-substituted alkyl, alkoxy, alkylthio, alkylsulphinyl, alkylsulphonyl, alkylamino, alkylcarbonyl, alkoxycarbonyl, alkylaminocarbonyl, alkylcarbonylamino, alkoxycarbonylamino, alkylsulphonylamino or bis-alkylsulphonyl-amino having in each case up to 6 carbon atoms,
R⁸ represents optionally halogen-substituted alkyl having 1 to 6 carbon atoms, represents optionally halogen-substituted alkenyl having 2 to 6 carbon atoms, represents optionally cyano-, halogen- or C₁-C₄-alkyl-substituted cycloalkyl having 3 to 6 carbon atoms, represents in each case optionally nitro-, cyano-, thiocarbamoyl-, halogen-, C₁-C₄-alkyl, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulphinyl-, C₁-C₄-halogenoalkylsulphinyl-, C₁-C₄-alkylsulphonyl-, C₁-C₄-halogenoalkylsulphonyl- or C₁-C₄-alkoxy-carbonyl-substituted aryl or arylalkyl having in each case 6 or 10 carbon atoms in the aryl group and optionally 1 to 4 carbon atoms in the alkyl moiety, or represents optionally cyano-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulphinyl-, C₁-C₄-halogenoalkylsulphinyl-, C₁-C₄-alkylsulphonyl- or C₁-C₄-halogenoalkylsulphonyl-substituted heterocyclyl from the series consisting of furyl, thienyl, oxazolyl, isoxazolyl, pyrazolyl, pyridyl, pyrimidyl, and
R⁹ represents hydrogen, represents in each case optionally cyano-, halogen- or C₁-C₄-alkoxy-substituted alkyl, alkoxy, alkylamino or dialkylamino having in each case 1 to 6 carbon atoms, represents in each case optionally cyano- or halogen-substituted alkenyl or alkinyl having in each case 2 to 6 carbon atoms, represents in each case optionally cyano-, halogen- or C₁-C₄-alkyl-substituted cycloalkyl or cycloalkylalkyl having in each case 3 to 6 carbon atoms in the cycloalkyl group and optionally 1 to 4 carbon atoms in the alkyl moiety, represents in each case optionally nitro-, cyano-, thiocarbamoyl-, halogen-, C₁-C₄-alkyl-, C₁-C₄-halogenoalkyl-, C₁-C₄-alkoxy-, C₁-C₄-halogenoalkoxy-, C₁-C₄-alkylthio-, C₁-C₄-halogenoalkylthio-, C₁-C₄-alkylsulphinyl-, C₁-C₄-halogenoalkylsulphinyl-, C₁-C₄-alkylsulphonyl-, C₁-C₄-halogenoalkylsulphonyl- or C₁-C₄-alkoxy-carbonyl-substituted aryl or arylalkyl having in each case 6 or 10 carbon atoms in the aryl group and optionally 1 to 4 carbon atoms in the alkyl moiety, or represents optionally cyano-, halogen-, C₁-C₄-alkyl-, C₁-C₄-halogenoalkyl-, C₁-C₄-alkoxy-, C₁-C₄-halogeno-alkoxy-, C₁-C₄-alkylthio-, C₁-C₄-halogenoalkylthio-, C₁-C₄-alkylsulphinyl-, C₁-C₄-halogenoalkylsulphinyl-, C₁-C₄-alkylsulphonyl- or C₁-C₄-halogenoalkylsulphonyl-substituted heterocyclyl from the series consisting of furyl, thienyl, oxazolyl, isoxazolyl, pyrazolyl, pyridyl, pyrimidyl,
except for the compounds 1-[4-(4-nitro-phenylmethoxy-phenyl)]-3-methyl-4-trifluoromethyl-3,6-dihydro-2,6-dioxo-1(2H)-pyrimidine and 1-[2-fluoro-4-(4-nitro-phenylmethoxy-phenyl)]-3-methyl-4-trifluoromethyl-3,6-dihydro-2,6-dioxo-1(2H)-pyrimidine.

2. Substituted phenyluracils according to Claim 1, **characterized in that**
m represents the numbers 0, 1 or 2,
n represents the numbers 0, 1 or 2,
Q represents O, S, SO, SO₂, NH or N(CH₃),
R¹ represents hydrogen, amino or represents in each case optionally cyano-, fluorine-, chlorine-, methoxy- or ethoxy-substituted methyl, ethyl, n- or i-propyl, n-, i-, s- or t-butyl,
R² represents carboxyl, cyano, carbamoyl, thiocarbamoyl, or represents in each case optionally cyano-, fluorine-, chlorine-, methoxy- or ethoxy-substituted methyl, ethyl, n- or i-propyl, n-, i-, s- or t-butyl, methoxycarbonyl, ethoxycarbonyl, n- or i-propoxycarbonyl,
R³ represents hydrogen, fluorine, chlorine, bromine, or represents in each case optionally cyano-, fluorine-, chlorine-, methoxy- or ethoxy-substituted methyl, ethyl, n- or i-propyl, n-, i-, s- or t-butyl,
R⁴ represents hydrogen, cyano, fluorine or chlorine,
R⁵ represents nitro, amino, hydroxyl, carboxyl, cyano, carbamoyl, thiocarbamoyl, sulpho, chlorosulphonyl, aminosulphonyl, fluorine, chlorine, bromine, or represents in each case optionally cyano-, fluorine-, chlorine-, methoxy- or ethoxy-substituted methyl, ethyl, n-or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, acetyl, propionyl, n- or i-butyroyl, methoxycarbonyl, ethoxycarbonyl, n-or i-propoxycarbonyl, methylaminocarbonyl, ethylaminocarbonyl, n-or i-propylaminocarbonyl, acetylamino, propionylamino, n- or i-butyroylamino, methoxycarbonylamino, ethoxycarbonylamino, n- or i-propoxycarbonylamino, methylsulphonylamino, ethylsulphonyl-amino, n- or i-propylsulphonylamino, n-, i-, s- or t-butylsulphonylamino, and
R⁶ represents nitro, amino or represents one of the groupings below
-NH-R⁸, -N(R⁸)₂, -NH-SO₂-R⁸, -N(R⁸)(SO2-R⁸), -N(SO₂-R⁸)₂,
-NH-CO-R⁹, -N(R⁸)(CO-R⁹), -N(CO-R⁹)₂, -N(SO₂-R⁸)(CO-R⁹),
R⁷ represents nitro, amino, hydroxyl, mercapto, carboxyl, cyano, carbamoyl, thiocarbamoyl, sulpho, chlorosulphonyl, aminosulphonyl, fluorine, chlorine, bromine, or represents in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, acetyl, propionyl, n- or i-butyroyl, methoxycarbonyl, ethoxycarbonyl, n- or i-propoxycarbonyl, methylaminocarbonyl, ethylaminocarbonyl, n- or i-propylaminocarbonyl, acetylamino, propionylamino, n- or i-butyroylamino, methoxycarbonylamino, ethoxycarbonylamino, n- or i-propoxycarbonylamino, methylsulphonylamino, ethylsulphonylamino, n- or i-propylsulphonylamino, n-, i-, s- or t-butylsulphonylamino, bis-(methylsulphonyl)-amino or bis-(ethylsulphonyl)-amino,
R⁸ represents in each case optionally fluorine and/or chlorine-substituted methyl, ethyl, n- or i-propyl, n-, i-, s- or t-butyl, represents in each case optionally fluorine- and/or chlorine-substituted ethenyl, propenyl or butenyl, represents in each case optionally cyano-, fluorine-, chlorine-, methyl- or ethyl-substituted cyclopropyl, cyclobutyl, cyclopentyl or cyclohexyl, represents in each case optionally nitro-, cyano-, thiocarbamoyl-, fluorine-, chlorine-, bromine-, methyl-, ethyl-, n- or i-propyl-, n-, i-, s- or t-butyl-, trifluoromethyl-, methoxy-, ethoxy-, n-or i-propoxy-, difluoromethoxy-, trifluoromethoxy-, methylthio-, ethylthio-, n- or i-propylthio-, difluoromethylthio-, trifluoromethylthio-, methylsulphinyl-, ethylsulphinyl-, trifluoromethylsulphinyl-, methylsulphonyl-, ethylsulphonyl-, trifluoromethylsulphonyl-, methoxycarbonyl- or ethoxy-carbonyl-substituted phenyl or benzyl, or represents optionally cyano-, fluorine-, chlorine-, bromine-, methyl-, ethyl-, n- or i-propyl-, n-, i-, s- or t-butyl, trifluoromethyl-, methoxy-, ethoxy-, n- or i-propoxy-, n-, i-, s- or t-butoxy-, difluoromethoxy-, trifluoromethoxy-, methylthio-, ethylthio-, n- or i-propylthio-, n-, i-, s-or t-butylthio-, trifluoromethylthio-, methylsulphinyl-, ethylsulphinyl-, trifluoromethylsulphinyl-, methylsulphonyl-, ethylsulphonyl-, or trifluoromethylsulphonyl-substituted heterocyclyl from the series consisting of furyl, thienyl, oxazolyl, isoxazolyl, pyrazolyl, pyridyl, pyrimidyl, and
R⁹ represents hydrogen, represents in each case optionally cyano-, fluorine-, chlorine-, methoxy- or ethoxy-substituted methyl, ethyl, n-or i-propyl, n-, i-, s- or t-butyl, methoxy, ethoxy, n- or i-propoxy, n-, i-, s- or t-butoxy, methylamino, ethylamino, n- or i-propylamino, n-, i-, s- or t-butylamino or dimethylamino, represents in each case optionally cyano-, fluorine- or chlorine-substituted ethenyl, propenyl, butenyl, ethinyl, propinyl or butinyl, represents in each case optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents in each case optionally nitro-, cyano-, thiocarbamoyl-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, n-, i-, s- or t-butoxy-, difluoromethoxy-, trifluoromethoxy-, methylthio-, ethylthio-, n- or i-propylthio-, difluoromethylthio-, trifluoromethylthio-, methylsulphinyl-, ethylsulphinyl-, trifluoromethylsulphinyl-, methylsulphonyl-, ethylsulphonyl-, trifluoromethylsulphonyl-, methoxycarbonyl-, ethoxycarbonyl-, n- or i-propoxy-carbonyl-substituted phenyl or benzyl, or represents optionally cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, n-, i-, s- or t-butoxy-, difluoromethoxy-, trifluoromethoxy-, methylthio-, ethylthio-, n- or i-propylthio-, trifluoromethylthio-, methylsulphinyl-, ethylsulphinyl-, trifluoromethylsulphinyl-, methylsulphonyl-, ethyl-sulphonyl- or trifluoromethylsulphonyl-substituted heterocyclyl from the series consisting of furyl, thienyl, oxazolyl, isoxazolyl, pyrazolyl, pyridyl, pyrimidyl,
except for the compounds 1-[4-(4-nitro-phenylmethoxy-phenyl)]-3-methyl-4-trifluoromethyl-3,6-dihydro-2,6-dioxo-1(2H)-pyrimidine and 1-[2-fluoro-4-(4-nitro-phenylmethoxy-phenyl)]-3-methyl-4-trifluoromethyl-3,6-dihydro-2,6-dioxo-1(2H)-pyrimidine.

3. Process for preparing substituted phenyluracils of the formula (I) according to Claim 1 or 2,
in which
m, n, R¹, R², R³, R⁴, R⁵, R⁶ and R⁷ have the meaning given in Claim 1 or 2, and Q represents O, S, NH or N(C₁-C₄-alkyl), **characterized in that**
(a) phenyluracils of the general formula (II) 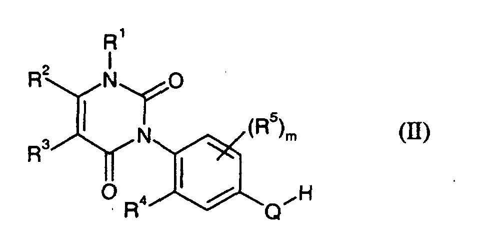
in which
m, R¹, R², R³, R⁴ and R⁵ are each as defined in Claim 1 or 2, and Q represents O, S, NH or N(C₁-C₄-alkyl),
are reacted with substituted benzyl halides of the general formula (III) 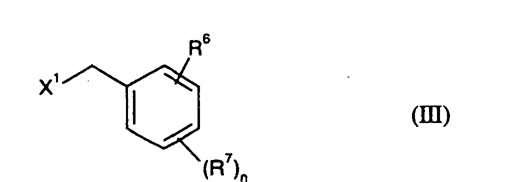 in which
n, R⁶ and R⁷ are each as defined in Claim 1 or 2 and
X¹ represents halogen,
if appropriate in the presence of a reaction auxiliary and if appropriate in the presence of a diluent,
or **in that**
(b) substituted phenyluracils of the general formula (Ia) 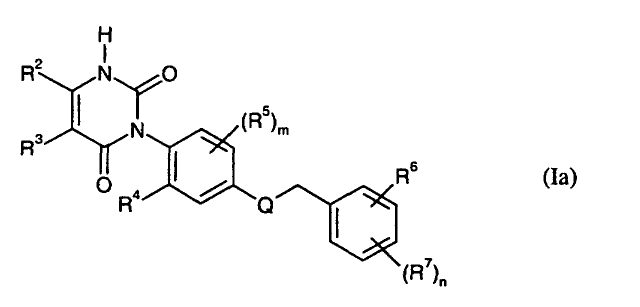
in which
m, n, Q, R², R³, R⁴, R⁵, R⁶ and R⁷ are each as defined above,
are reacted with 1-aminooxy-2,4-dinitro-benzene or with alkylating agents of the general formula (IV)
X²-A¹ (IV)
in which
A¹ represents optionally substituted C₁-C₆-alkyl and
X² represents halogen or the grouping -O-SO₂-O-A¹,
if appropriate in the presence of a reaction auxiliary and if appropriate in the presence of a diluent,
and, if appropriate, electrophilic or nucleophilic and/or oxidation or reduction reactions within the scope of the definition of the substituents are carried out subsequently in a customary manner.

4. Substituted phenyluracils of the general formula (Ia) 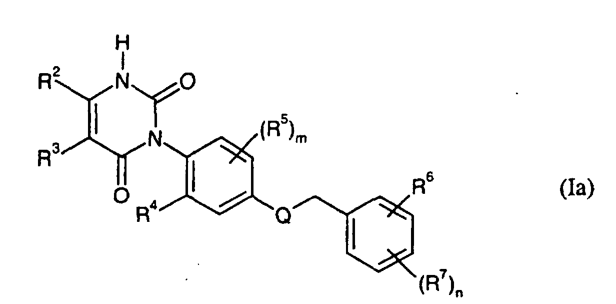 in which
m, n, Q, R², R³, R⁴, R⁵, R⁶ and R⁷ are each as defined in Claim 1 or 2.

5. Use of at least one substituted phenyluracil according to Claim 1 or 2 for controlling undesirable plants.

6. Herbicidal composition, **characterized in that** it comprises at least one substituted phenyluracil according to Claim 1 or 2 and customary extenders.

## Revendications

1. Phényluraciles substitués de formule générale (I) 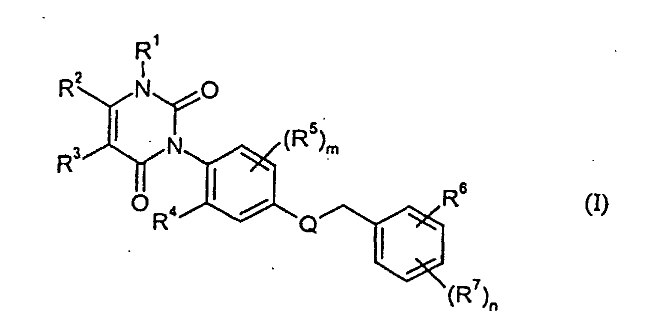 dans laquelle
m représente les nombres 0, 1 ou 2,
n représente les nombres 0, 1, 2 ou 3,
Q représente O, S, SO, SO₂, NH ou un reste N(alkyle en C₁ à C₄),
R¹ est l'hydrogène, un groupe amino ou un reste alkyle ayant 1 à 6 atomes de carbone, substitué par un radical cyano, halogéno ou alkoxy en C₁ à C₄,
R² est un reste carboxy, cyano, carbamoyle, thiocarbamoyle ou un reste alkyle ou alkoxycarbonyle ayant chacun jusqu'à 6 atomes de carbone et portant chacun, le cas échéant, un substituant cyano, halogéno ou alkoxy en C₁ à C₄,
R³ est l'hydrogène, un halogène ou un reste alkyle ayant 1 à 6 atomes de carbone éventuellement substitué par un radical cyano, halogéno ou alkoxy en C₁ à C₄,
R⁴ est l'hydrogène, un reste cyano ou un halogène,
R⁵ est un reste nitro, amino, hydroxy, carboxy, cyano, carbamoyle, thiocarbamoyle, sulfo, chlorosulfonyle, aminosulfonyle, halogéno, ou un reste alkyle, alkoxy, alkylthio, alkylsulfinyle, alkylsulfonyle, alkylamino, alkylcarbonyle, alkoxycarbonyle, alkylaminocarbonyle, alkylcarbonylamino, alkoxycarbonylamino ou alkylsulfonylamino ayant chacun jusqu'à 6 atomes de carbone et portant chacun un substituant cyano, halogéno ou alkoxy en C₁ à C₄, et
R⁶ est un groupe amino ou l'un des groupements suivants :
-NH-R⁸, -N(R⁸)₂, -NH-SO₂-R⁸, -N(R⁸)(SO2-R⁸), -N(SO₂-R⁸)₂,
-NH-CO-R⁹, -N(R⁸)(CO-R⁹), -N(CO-R⁹)₂, -N(SO₂-R⁸)(CO-R⁹)
R⁷ est un reste nitro, amino, hydroxy, mercapto, carboxy, cyano, carbamoyle, thiocarbamoyle, sulfo, chloresulfonyle, aminosulfonyle, halogéno, ou un reste alkyle, alkoxy, alkylthio, alkylsulfinyle, alkylsulfonyle, alkylamino, alkylcarbonyle, alkoxycarbonyle, alkylaminocarbonyle, alkylcarbonylamino, alkoxycarbonylamino, alkylsulfonylamino ou bis-alkylsulfonylamino ayant chacun jusqu'à 6 atomes de carbone et portant chacun, le cas échéant, un substituant cyano, halogéno ou alkoxy en C₁ à C₄,
R⁸ est un reste alkyle ayant 1 à 6 atomes de carbone éventuellement substitué par un halogène, un reste alcényle ayant 2 à 6 atomes de carbone éventuellement substitué par un halogène, un reste cycloalkyle ayant 3 à 6 atomes de carbone éventuellement substitué par un radical cyano, halogéno ou alkyle en C₁ à C₄, un reste aryle ou arylalkyle ayant chacun 6 ou 10 atomes de carbone dans le groupe aryle et, le cas échéant, 1 à 4 atomes de carbone dans la partie alkyle, chacun étant éventuellement substitué par un radical nitro, cyano, thiocarbamoyle, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄ ou (alkoxy en C₁ à C₄)-carbonyle, ou un reste hétérocyclyle de la série furyle, thiényle, oxazolyle, isoxazolyle, pyrazolyle, pyridyle ou pyrimidyle éventuellement substitué par un radical cyano, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄ ou halogénalkylsulfonyle en C₁ à C₄, et
R⁹ représente l'hydrogène, un reste alkyle, alkoxy, alkylamino ou dialkylamino ayant chacun 1 à 6 atomes de carbone et portant chacun, le cas échéant, un substituant cyano, halogéno ou alkoxy en C₁ à C₄, un reste alcényle ou alcynyle ayant chacun 2 à 6 atomes de carbone et portant chacun, le cas échéant, un substituant cyano ou halogéno, un reste cycloalkyle ou cycloalkylalkyle ayant chacun 3 à 6 atomes de carbone dans le groupe cycloalkyle et, le cas échéant, 1 à 4 atomes de carbone dans la partie alkyle, portant chacun, le cas échéant, un substituant cyano, halogéno
ou alkyle en C₁ à C₄, un reste aryle ou arylalkyle ayant chacun 6 ou 10 atomes de carboné dans la partie aryle et, le cas échéant, 1 à 4 atomes de carbone dans la partie alkyle et portant chacun, le cas échéant, un substituant nitro, cyano, thiocarbamoyle, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄, halogénalkylsulfonyle en C₁ à C₄ ou (alkoxy en C₁ à C₄)-carbonyle, ou bien un reste hétérocyclyle de la série furyle, thiényle, oxazolyle, isoxazolyle, pyrazolyle, pyridyle, pyrimidyle, portant chacun, le cas échéant, un substituant cyano, halogéno, alkyle en C₁ à C₄, halogénalkyle en C₁ à C₄, alkoxy en C₁ à C₄, halogénalkoxy en C₁ à C₄, alkylthio en C₁ à C₄, halogénalkylthio en C₁ à C₄, alkylsulfinyle en C₁ à C₄, halogénalkylsulfinyle en C₁ à C₄, alkylsulfonyle en C₁ à C₄ ou halogénalkylsulfonyle en C₁ à C₄,
à l'exception des composés 1-[4-(4-nitrophénylméthoxy-phényl)]-3-méthyl-4-trifluorométhyl-3,6-dihydro-2,6-dioxo-1(2H)-pyrimidine et 1- [2-fluoro-4- (4-nitrophénylméthoxy-phényl)]-3-méthyl-4-trifluorométhyl-3,6-dihydro-2,6-dioxo-1(2H)-pyrimidine.

2. Phényluraciles substitués suivant la revendication 1, **caractérisés en ce que**
m représente les nombres 0, 1 ou 2,
n représente les nombres 0, 1 ou 2,
Q représente O, S, SO, SO₂, NH ou N(CH₃),
R¹ est l'hydrogène, un groupe amino ou un reste méthyle, éthyle, n-propyle, isopropyle, n-butyle, isobutyle, sec.-butyle ou tertio-butyle portant chacun, le cas échéant, un substituant cyano, fluoro, chloro, méthoxy ou éthoxy,
R² est un reste carboxy, cyano, carbamoyle, thiocarbamoyle ou un reste méthyle, éthyle, n-propyle, isopropyle, n-butyle, isobutyle, sec.-butyle, tertiobutyle, méthoxycarbonyle, éthoxycarbonyle, n-propoxycarbonyle ou isopropoxycarbonyle portant chacun, le cas échéant, un substituant cyano, fluoro, chloro, méthoxy ou éthoxy,
R³ est l'hydrogène, le fluor, le chlore, le brome ou un reste méthyle, éthyle, n-propyle, isopropyle, n-butyle, isobutyle, sec.-butyle ou tertio-butyle portant chacun, le cas échéant, un substituant cyano, fluoro, chloro, méthoxy ou éthoxy,
R⁴ est l'hydrogène, un reste cyano, fluoro ou chloro,
R⁵ est un reste nitro, amino, hydroxy, carboxy, cyano, carbamoyle, thiocarbamoyle, sulfo, chlorosulfonyle, aminosulfonyle, le fluor, le chlore, le brome ou un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylsulfinyle, éthylsulfinyle, n-propylsulfinyle, isopropylsulfinyle, méthylsulfonyle, éthylsulfonyle, n-propylsulfonyle, isopropylsulfonyle, méthylamino, éthylamino, n-propylamino, isopropylamino, n-butylamino, isobutylamino, sec.-butylamino, tertio-butylamino, acétyle, propionyle, n-butyroyle, isobutyroyle, méthoxycarbonyle, éthoxycarbonyle, n-propoxycarbonyle, isopropoxycarbonyle, méthylaminocarbonyle, éthylaminocarbonyle, n-propylaminocarbonyle, isopropylaminocarbonyle, acétylamino, propionylamino, n-butyroylamino, isobutyroylamino, méthoxycarbonylamino, éthoxycarbonylamino, n-propoxycarbonylamino, isopropoxycarbonylamino, méthylsulfonylamino, éthylsulfonylamino, n-propylsulfonylamino, isopropylsulfonylamino, n-butylsulfonylamino, isobutylsulfonylamino, sec.-butylsulfonylamino ou tertio-butylsulfonylamino portant chacun, le cas échéant, un substituant cyano, fluoro, chloro, méthoxy ou éthoxy, et
R⁶ est un groupe amino ou l'un des groupements suivants :
-NH-R⁸, -N(R⁸)₂, -NH-SO₂-R⁸, -N(R⁸)(SO2-R⁸), -N(SO₂-R⁸)₂,
-NH-CO-R⁹, -N(R⁸)(CO-R⁹),-N(CO-R⁹)₂,-N(SO₂-R⁸)(CO-R⁹)
R⁷ est un reste nitro, amino, hydroxy, mercapto, carboxy, cyano, carbamoyle, thiocarbamoyle, sulfo, chlorosulfonyle, aminosulfonyle, le fluor, le chlore, le brome, ou bien un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertio-butoxy, méthylthio, éthylthio, n-propylthio, isopropylthio, n-butyl-thio, isobutylthio, sec.-butylthio, tertio-butylthio, méthylsulfinyle, éthylsulfinyle, n-propylsulfinyle, isopropylsulfinyle, méthylsulfonyle, éthylsulfonyle, n-propylsulfonyle, isopropylsulfonyle, méthylamino, éthylamino, n-propylamino, isopropylamino, n-butylamino, isobutylamino, sec.-butylamino, tertio-butylamino, acétyle, propionyle, n-butyroyle, isobutyroyle, méthoxycarbonyle, éthoxycarbonyle, n-propoxycarbonyle, isopropoxycarbonyle, méthylaminocarbonyle, éthylaminocarbonyle, n-propylaminocarbonyle, isopropylaminocarbonyle, acétylamino, propionylamino, n-butyroylamino, isobutyroylamino, méthoxycarbonylamino, éthoxycarbonylamino, n-propoxycarbonylamino, isopropoxycarbonylamino, méthylsulfonylamino, éthylsulfonylamino, n-propylsulfonylamino, isopropylsulfonylamino, n-butylsulfonylamino, isobutylsulfonylamino, sec.-butylsulfonylamino, tertio-butylsulfonylamino, bis-(méthylsulfonyl)-amino ou bis-(éthylsulfonyl)-amino, portant chacun, le cas échéant, un substituant cyano, fluoro, chloro, méthoxy ou éthoxy,
R⁸ est un reste méthyle, éthyle, n-propyle, isopropyle, n-butyle, isobutyle, sec.-butyle, tertio-butyle portant chacun, le cas échéant, un substituant fluoro et/ou chloro, un reste éthényle, propényle ou butényle portant chacun, le cas échéant, un substituant fluoro et/ou chloro, un reste cyclopropyle, cyclobutyle, cyclopentyle ou cyclohexyle portant chacun, le cas échéant, un substituant cyano, fluoro, chloro, méthyle ou éthyle, un reste phényle ou benzyle portant chacun, le cas échéant, un substituant nitro, cyano, thio-carbamoyle, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, trifluorométhoxy, méthylthio, éthylthio, n-propylthio, isopropylthio, difluorométhylthio, trifluorométhylthio, méthylsulfinyle, éthylsulfinyle, trifluorométhylsulfinyle, méthylsulfonyle, éthylsulfonyle, trifluorométhylsulfonyle, méthoxycarbonyle ou éthoxycarbonyle, ou bien un reste hétérocyclyle de la série furyle, thiényle, oxazolyle, isoxazolyle, pyrazolyle, pyridyle, pyrimidyle portant, le cas échéant, un substituant cyano, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, n-butoxy, isobutoxy, sec.-butoxy, tertio-butoxy, difluorométhoxy, trifluorométhoxy, méthylthio, éthylthio, n-propylthio, isopropylthio, n-butylthio, isobutylthio, sec.-butylthio, tertio-butylthio, trifluorométhylthio, méthylsulfinyle, éthylsulfinyle, trifluorométhylsulfinyle, méthylsulfonyle, éthylsulfonyle ou trifluorométhylsulfonyle, et
R⁹ représente l'hydrogène, un reste méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylamino, éthylamino, n-propylamino, isopropylamino, n-butylamino, isobutylamino, sec.-butylamino, tertio-butylamino ou diméthylamino portant chacun, le cas échéant, un substituant cyano, fluoro, chloro, méthoxy ou éthoxy, un reste éthényle, propényle, butényle, éthynyle, propynyle ou butynyle portant chacun, le cas échéant, un substituant cyano, fluoro ou chloro, un reste cyclopropyle, cyclobutyle, cyclopentyle, cyclohexyle, cyclopropylméthyle, cyclobutylméthyle, cyclpentylméthyle ou cyclohexylméthyle portant chacun, le cas échéant, un substituant cyano, fluoro, chloro, méthyle ou éthyle, un reste phényle ou benzyle portant chacun, le cas échéant, un substituant nitro, cyano, thiocarbamoyle, fluoro, chloro, bromo, méthyle, éthyle, n-propyle, isopropyle, n-butyle, iso-butyle, sec.-butyle, tertio-butyle, trifluorométhyle, méthoxy, éthoxy, n-propoxy, isopropoxy, n-butoxy, isobutoxy, sec.-butoxy, tertio-butoxy, difluorométhoxy, trifluorométhoxy, méthylthio, éthylthio, n-propylthio, isopropylthio, difluorométhylthio, trifluorométhylthio, méthylsulfinyle, éthylsulfinyle, trifluorométhylsulfinyle, méthylsulfonyle, éthylsulfonyle, trifluorométhylsulfonyle, méthoxycarbonyle, éthoxycarbonyle, n-propoxycarbonyle ou isopropoxycarbonyle, ou bien un reste hétérocyclyle de la série furyle, thiényle, oxazolyle, isoxazolyle, pyrazolyle, pyridyle, pyrimidyle portant chacun, le cas échéant, un substituant cyano, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, n-butoxy, isobutoxy, sec.-butoxy, tertio-butoxy, difluorométhoxy, trifluorométhoxy, méthylthio, éthylthio, n-propylthio, isopropylthio, trifluorométhylthio, méthylsulfinyle, éthylsulfinyle, trifluorométhylsulfinyle, méthylsulfonyle, éthylsulfonyle ou trifluorométhylsulfonyle,
à l'exception des composés 1-[4-(4-nitrophénylméthoxy-phényl)]-3-méthyl-4-trifluorométhyl-3,6-dihydro-2,6-dioxo-1(2H)-pyrimidine et 1-[2-fluoro-4-(4-nitrophénylméthoxy-phényl)]-3-méthyl-4-trifluorométhyl-3,6-dihydro-2,6-dioxo-1(2H)-pyrimidine.

3. Procédé de production de phényluraciles substitués de formule (I) suivant l'une des revendications 1 et 2, formule dans laquelle
m, n, R¹, R², R³, R⁴, R⁵, R⁶ et R⁷ ont la définition indiquée dans l'une des revendications 1 ou 2 et Q représente O, S, NH ou un reste N(alkyle en C₁ à C₄),
**caractérisé en ce que** :
(a) on fait réagir des phényluraciles de formule générale (II) 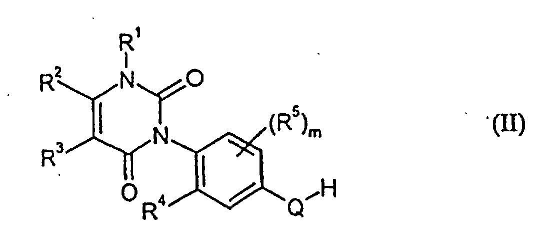
dans laquelle
m, R¹, R², R³, R⁴ et R⁵ ont la définition indiquée dans l'une des revendications 1 ou 2 et Q représente O, S, NH ou un reste N(alkyle en C₁ à C₄)
avec des halogénures de benzyle substitués de formule générale (III) 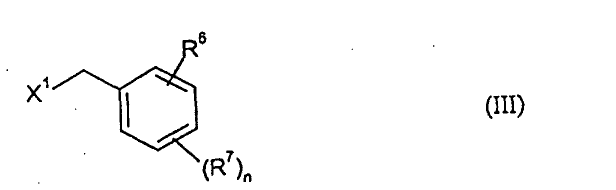 dans laquelle
n, R⁶ et R⁷ ont la définition indiquée dans l'une des revendications 1 ou 2 et
X¹ est un halogène,
le cas échéant en présence d'un auxiliaire de réaction et en présence éventuelle d'un diluant,
ou **en ce que**
(b) on fait réagir des phényluraciles substitués de formule générale (Ia) 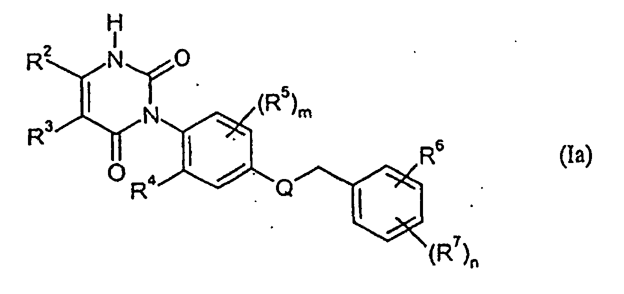
dans laquelle
m, n, Q, R², R³, R⁴, R⁵, R⁶ et R⁷ ont la définition indiquée ci-dessus,
avec le 1-amino-oxy-2,4-dinitrobenzène ou avec des agents d'alkylation de formule générale (IV)
X²-A¹ (IV)
dans laquelle
A¹ est un reste alkyle en C₁ à C₆ éventuellement substitué et
X² est un halogène ou le groupement -O-SO₂-O-A¹,
le cas échéant en présence d'un auxiliaire de réaction et en présence éventuelle d'un diluant,
après quoi, le cas échéant, on conduit d'une manière classique des réactions électrophiles ou nucléophiles, autrement dit d'oxydation ou de réduction dans le cadre de la définition des substituants.

4. Phényluraciles substitués de formule générale (Ia) 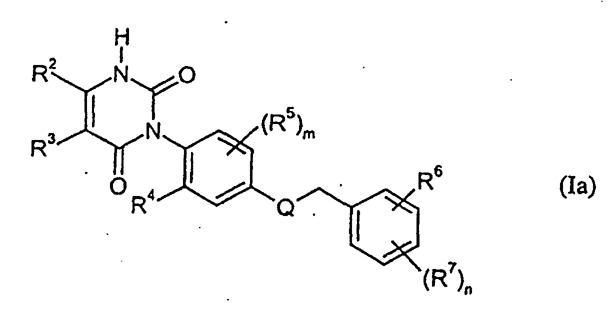 dans laquelle
m, n, Q, R², R³, R⁴, R⁵, R⁶ et R⁷ ont la définition indiquée dans l'une des revendications 1 ou 2.

5. Utilisation d'au moins un phényluracile substitué suivant l'une des revendications 1 ou 2 pour combattre des plantes indésirables.

6. Composition herbicide, **caractérisée par** une teneur en au moins un phényluracile substitué suivant l'une des revendications 1 ou 2 et des diluants classiques.
